(19) Europäisches Patentamt / European Patent Office / Office européen des brevets

(11) **EP 2 552 358 B1**

(12) **EUROPÄISCHE PATENTSCHRIFT**

(45) Veröffentlichungstag und Bekanntmachung des Hinweises auf die Patenterteilung:
**18.05.2016  Patentblatt 2016/20**

(21) Anmeldenummer: **11712543.5**

(22) Anmeldetag: **31.03.2011**

(51) Int Cl.:
*A61F 2/38* *(2006.01)*      *A61F 2/00* *(2006.01)*
*A61F 2/30* *(2006.01)*

(86) Internationale Anmeldenummer:
**PCT/EP2011/055042**

(87) Internationale Veröffentlichungsnummer:
**WO 2011/121088 (06.10.2011 Gazette 2011/40)**

(54) **KNIEGELENK MIT VIERGELENKSFUNKTION UND SCHARNIERFORTSETZUNG**

KNEE JOINT WITH FOUR-JOINT FUNCTION AND HINGE CONTINUATION

ARTICULATION DU GENOU AYANT UNE FONCTION ARTICULATION QUADRUPLE ET UN PROLONGEMENT DE CHARNIÈRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.04.2010  DE 102010016308**

(43) Veröffentlichungstag der Anmeldung:
**06.02.2013  Patentblatt 2013/06**

(73) Patentinhaber: **Limacorporate S.p.A.**
**33038 San Daniele Del Friuli (UD) (IT)**

(72) Erfinder: **NÄGERL, Hans**
**37130 Gleichen-Klein Lengden (DE)**

(74) Vertreter: **Petraz, Gilberto Luigi et al**
**GLP S.r.l.**
**Viale Europa Unita, 171**
**33100 Udine (IT)**

(56) Entgegenhaltungen:
**EP-A1- 1 129 676          EP-A1- 1 970 030**
**EP-B1- 0 617 595          WO-A1-2011/029908**
**DE-A1- 19 521 597        DE-A1- 19 646 891**
**DE-C1- 10 231 538        US-A- 5 358 527**
**US-A1- 2010 191 341**

**Beschreibung**

[GEBIET DER ERFINDUNG]

Ausführungsformen der vorliegenden Erfindung beziehen sich auf ein mechanisches Gelenk, insbesondere auf eine Endoprothese für ein menschliches Gelenk, typischerweise ein menschliches Kniegelenk. Spezifischer beziehen sich einige Ausführungsformen auf ein mechanisches Gelenk mit Viergelenksfunktion in einem ersten Winkelbereich und Scharnierfunktion in einem zweiten Winkelbereich.

[HINTERGRUND DER ERFINDUNG]

Durch Verletzung oder Abnutzung können Gelenke von Lebewesen in einer Weise beeinträchtigt sein, dass sie ihre natürliche Funktion nicht mehr ausüben können. Als Ersatz sind künstliche, mechanische Gelenke bekannt, die oftmals als Endoprothese in den Körper implantiert werden.

Aus EP 0617595 ist ein Kniegelenk gemäss des Oberbegriffs von Anspruch 1 bekannt.

Solche mechanischen Gelenke sollen im Idealfall die Funktionen des natürlichen Gelenks für den gesamten Winkelbereich nachbilden, d.h. von Streckung oder gar Überstreckung bis zur maximalen Beugung, und dabei mit dem Muskelapparat in einer Weise zusammenarbeiten, dass erlernte Muskelkontrolle auch weiterhin dieselben Gelenkbewegungen auslöst. Zu berücksichtigen sind dabei jedoch die Möglichkeiten von Werkstoffen der mechanischen Gelenke, die in aller Regel einen "Eins-zu-eins" Nachbau der Natur nicht gestatten. Bekannte mechanische Gelenke leisten dies jedoch nicht immer in hinreichender Weise. So sind beispielsweise künstliche Kniegelenke bekannt, die die natürliche Kniekinematik bei kleinen Beugungswinkeln gut widerspiegelt wie sie beim Gehen oder Laufen auftreten. Kleine Beugungswinkel können dabei z.B. Winkel von 0° (gestrecktes Gelenk wie im Stand) bis 30° (leichte Beugung) sein. Bei großen Beugungswinkeln hingegen, wie sie z.B. beim Radfahren mit tiefem Sattel auftreten können, weicht die Kinematik vom natürlichen Verhalten ab. Bei solchen Prothesen darf beispielsweise der Sattel für das Radfahren nicht zu tief eingestellt werden. Auch eine Hocke ist nicht ohne Probleme für den betroffenen Menschen einzunehmen oder aufzulösen.

Somit besteht ein Bedarf, verbesserte mechanische Gelenke bereitzustellen, insbesondere Endoprothesen für menschliche Gelenke wie das Kniegelenk.

[ZUSAMMENFASSUNG DER ERFINDUNG]

Im Hinblick auf die zuvor genannten Probleme wird ein mechanisches Gelenk gemäß dem unabhängigen Anspruch 1 bereitgestellt. Weitere vorteilhafte Ausbildungen, die in geeigneter Weise beliebig miteinander kombiniert werden können, sind den abhängigen Ansprüchen, den Zeichnungen und der Beschreibung zu entnehmen.

Nach einer Ausführungsform wird ein mechanisches Gelenk, insbesondere eine Endoprothese für ein menschliches Gelenk bereitgestellt. Das mechanische Gelenk umfasst einen ersten Gelenkabschnitt mit einer ersten Gelenkfläche, einen ersten Gelenkgegenabschnitt mit einer ersten Gelenkgegenfläche, einen zweiten Gelenkabschnitt mit einer zweiten Gelenkfläche und einen zweiten Gelenkgegenabschnitt mit einer zweiten Gelenkgegenfläche. Die beiden Gelenkabschnitte sind zu einem ersten Gelenkteil und die beiden Gelenkgegenabschnitte zu einem zweiten Gelenkteil starr verbunden sind. Die erste Gelenkfläche und die erste Gelenkgegenfläche bilden durch Berührung an einem ersten Kontaktpunkt eine gelenkige erste Verbindung und die zweite Gelenkfläche und die zweite Gelenkgegenfläche bilden durch Berührung an einem zweiten Kontaktpunkt eine gelenkige zweite Verbindung, so dass der erste Gelenkteil relativ zum zweiten Gelenkteil eine Schwenkbewegung in einer Hauptfunktionsebene durchführen kann. In Bezug auf die Hauptfunktionsebene sind die Stärken der Krümmungen der ersten Gelenkfläche und der ersten Gelenkgegenfläche am ersten Kontaktpunkt durch erste Krümmungszentren und die Stärken der Krümmungen der zweiten Gelenkfläche und der zweiten Gelenkgegenfläche am zweiten Kontaktpunkt durch zweite Krümmungszentren beschrieben. Innerhalb der Hauptfunktionsebene fallen für Schwenkwinkel in einem ersten Winkelbereich die Krümmungszentren auseinander, wobei sie typischerweise ein Viereck bilden, und für Schwenkwinkel in einem zweiten Winkelbereich fallen mindestens zwei der Krümmungszentren zusammen, entsprechend der Definition in Anspruch 1.

Des Weiteren ist die Offenbarung auch auf Verfahren gerichtet, nach denen die beschriebenen mechanischen Gelenke arbeiten oder durch welche sie hergestellt werden. Diese Verfahren enthalten Verfahrensschritte zum Ausführen der Funktionen der Gelenke oder zur Herstellung dieser Funktionalität.

[KURZBESCHREIBUNG DER ABBILDUNGEN]

Einige der oben erwähnten und weitere detaillierte Aspekte werden in der Beschreibung näher bezeichnet und zumindest teilweise mit Bezug auf die Abbildungen erläutert.

| | |
|---|---|
| Fig. 1 | zeigt eine perspektivische Ansicht eines mechanischen Gelenks mit Viergelenksfunktion zum besseren Verständnis und/oder gemäß der hierin beschriebenen Ausführungsformen; |
| Figs. 2A und 2B | illustrieren das Roll-Gleitverhältnis. |
| Fig. 3 | zeigt eine laterale Gelenkfläche und Gelenkgegenfläche nebst Kontaktspur bei Schwenken des Gelenks |

gemäß hierin beschriebenen Ausführungsformen;

Fig. 4          illustriert die Projektion von Kontaktspuren in eine Ebene und stellt kine-
               matische Größen des mechani-
               schen Gelenks gemäß hierin be-
               schriebenen Ausführungsformen
               dar;

Figs. 5A bis 5E   zeigen sagittale Projektionen eines
                  mechanischen Kniegelenks gemäß
                  hierin beschriebenen Ausführungs-
                  formen nebst geometrischen Grö-
                  ßen;

Fig. 6          zeigt eine sagittale Projektion eines
               mechanischen Kniegelenks gemäß
               hierin beschriebenen Ausführungs-
               formen nebst kinematischen Grö-
               ßen;

Figs. 7 und 8   zeigen sagittale Projektionen eines
                mechanischen Kniegelenks gemäß
                hierin beschriebenen Ausführungsformen nebst geometrischen, bzw.
                kinematischen Größen bei verschiedenen Beugungszuständen.

## [DETAILLIERTE BESCHREIBUNG]

Innerhalb von Beschreibungen der Abbildungen beziehen sich gleiche Referenzzeichen auf gleiche
oder ähnliche Komponenten. Im Allgemeinen werden nur
die Unterschiede zwischen einzelnen Ausführungsformen beschrieben. Die Abbildungen sind nicht notwendigerweise maßstabsgetreu und dienen der Illustration.

Im Folgenden wird aus Gründen der einfacheren Verständlichkeit eine Endoprothese für ein menschliches Kniegelenk beschrieben. Entsprechend werden
Begriffe wie Femur (Oberschenkel, bzw. Oberschenkelknochen), femoral (zum Oberschenkel gehörig), Tibia
(Unterschenkel, bzw. Unterschenkelknochen) und tibial
(zum Unterschenkel gehörig) verwendet. Dies soll jedoch keine Einschränkung auf menschliche Kniegelenke
bedeuten. Die verbesserten mechanischen Gelenke
können z.B. auch Kniegelenke von Tieren ersetzen oder
gar andere Gelenke als ein Kniegelenk, z.B. ein Wirbelgelenk. Denkbar ist auch ein Einsatz bei Robotern. Für
solche anderen Gelenke sind die Begriffe ihren jeweiligen Entsprechungen gemäß zu ersetzen.

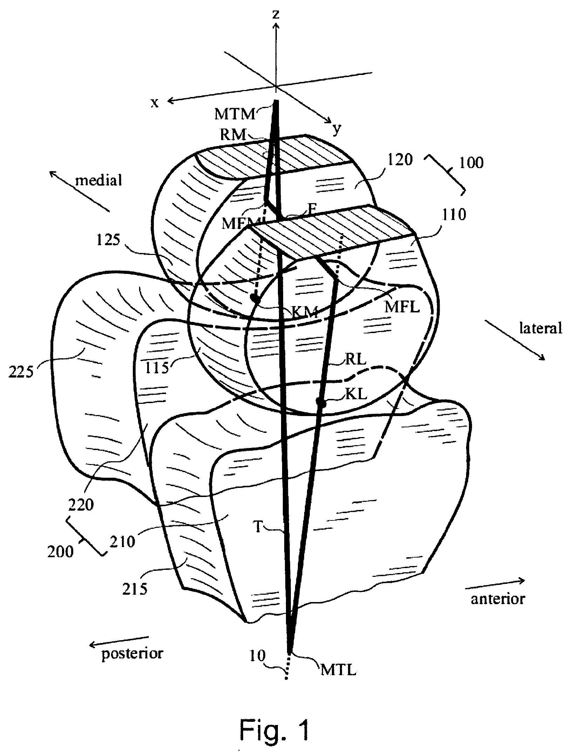

In Fig. 1 ist zur Orientierung ein x-y-z-Koordinatensystem gezeigt. Eine sagittale Ebene, d.h. eine von
unten noch oben und von vorne nach hinten durch den
Körper verlaufende Ebene, entspricht hier einer Ebene
parallel zur x-z-Ebene. In positiver x-Richtung verlaufend
ist hier mit "posterior" die Richtung nach hinten bezeichnet, d.h. nach dorsal, also zum Rücken hin. Entsprechend verläuft die negative x-Richtung nach anterior, d.h.
nach vorne zur Brust hin. Die mediale Richtung bezeichnet die Richtung senkrecht zu einer sagittalen Ebene zur
Innenseite des Knies hin, lateral die Richtung zur Außenseite des Knies. In Fig. 1 verläuft die mediale Richtung
in negativer y-Richtung, die laterale Richtung in positiver
y-Richtung. Es ist also das rechte Knie gezeigt. Für ein
linkes Knie wäre die gesamte Figur an einer sagittalen
Ebene zu spiegeln.

Fig. 1 zeigt eine perspektivische Ansicht eines
mechanischen Gelenks. Das mechanische Gelenk umfasst einen femoral-lateralen Gelenkabschnitt 110 und
einen femoral-medialen Gelenkabschnitt 120, die zu einem femoralen Gelenkteil 100 verbunden sind. Typischerweise ist die Verbindung zwischen den Gelenkteilen starr. Das mechanische Gelenk umfasst weiter einen
tibial-lateralen Gelenkgegenabschnitt 210 und einen tibial-medialen Gelenkgegenabschnitt 220, die zu einem
tibialen Gelenkteil 200 verbunden sind. Auch hier kann
die Verbindung zwischen den Gelenkteilen starr sein.

Der femoral-laterale Gelenkabschnitt weist eine Gelenkfläche 115 auf, der femoral-mediale Gelenkabschnitt 120 eine Gelenkfläche 125, der tibial-laterale Gelenkgegenabschnitt 210 eine Gelenkgegenfläche 215
und der tibial-mediale Gelenkgegenabschnitt 220 eine
Gelenkgegenfläche 225. Die Bezeichnung Gelenkfläche
und Gelenkgegenfläche, bzw. Gelenkabschnitt und Gelenkgegenabschnitt, ist wurde aus folgendem Grund gewählt. Jeweils eine Gelenkfläche 115, 125 des Femurgelenkteils 100 berührt die entsprechende Gelenkgegenfläche 215, 225 des Tibiagelenkteils 200. Dabei entsteht, typischerweise durch muskulären Kraftschluss, eine laterale gelenkige Verbindung zwischen dem femoral-
lateralen Gelenkabschnitt 110 und dem tibial-lateralen
Gelenkgegenabschnitt 210 und eine mediale gelenkige
Verbindung zwischen dem femoral-medialen Gelenkabschnitt 120 und dem tibial-medialen Gelenkgegenabschnitt 220. Ist nicht speziell von einem Gelenkflächenpaar, nämlich Fläche und Gegenfläche die Rede, so ist
unter dem allgemeinen Begriff "Gelenkfläche" auch eine
Gelenkgegenfläche zu verstehen.

In Fig. 1 berühren sich der femoral-laterale Gelenkabschnitt 110 und der tibial-laterale Gelenkgegenabschnitt 210 an einem lateralen Kontaktpunkt KL. Der
femoral-mediale Gelenkabschnitt 210 und der tibial-mediale Gelenkgegenabschnitt 220 berühren sich an einem
medialen Kontaktpunkt KM. Die Berührung erfolgt bei
einer implantierten Endoprothese typischerweise unter
Krafteinwirkung, d.h. Kraftschluss aufgrund von Gewichts- und/oder Muskelkräften. Man spricht dabei auch
von Artikulation, dem Ausbilden einer gelenkigen Verbindung durch das Zusammenwirken zwei aufeinander
abrollender/-gleitender Flächen.

Allgemein können gemäß einigen Ausführungsformen die Gelenkteile separierbar verbunden
sein. Der Zusammenschluss erfolgt wie oben beschrieben durch Kraftschluss. Die Verbindung erfolgt dann
nicht durch Elemente wie z.B. Bolzen, Scharnierbolzen,
Klammern oder Ähnlichem. Dies entspricht dem biologischen Vorbild zumindest beim Kniegelenk. Es werden
weitere Teile vermieden, die der Abnutzung unterliegen
können, in der Herstellung aufwendig und teuer sein können und/oder den natürlichen Bewegungsablauf stören. In anderen Ausführungsformen kann ein mechanisches Gelenk solche Verbindungselemente aufweisen. Dies kann z.B. erhöhter Stabilität dienen.

Die Lage der Kontaktpunkte hängt vom Beugungszustand ab. Generell kann sich die Lage der Kontaktpunkte verändern. Eine Veränderung der Lage der Kontaktpunkte entlang einer Kontaktspur erfolgt sowohl bei Gelenkfläche und Gelenkgegenfläche, wenn Gelenkfläche und Gelenkgegenfläche aufeinander rollen oder wenn die Bewegung zumindest einen Rollanteil hat. Es gibt zwei Arten des Gleitens: 1) Die Lage des Kontaktpunktes auf der Kontaktspur der Gelenkfläche bleibt unverändert, wenn die Gelenkgegenfläche auf der Gelenkfläche gleitet und deshalb der Kontakt nur auf der Gegengelenkfläche wandert. 2) Andersherum bleibt der Kontaktpunkte auf der Kontaktspur der Gelenkgegenfläche unverändert, wenn die Gelenkfläche auf der Gelenkgegenfläche gleitet. Allgemein kann bei einer Veränderung des Beugungszustands des Gelenks ein reines Rollen, ein reines Gleiten, oder oftmals gleichzeitig und anteilig ein Rollen und Gleiten erfolgen. Das Verhältnis der Anteile von Rollen und Gleiten wird Roll-Gleit-Verhältnis genannt.

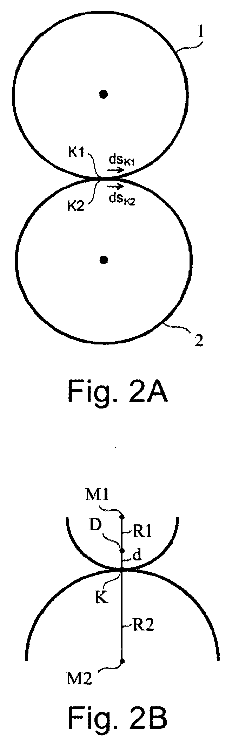

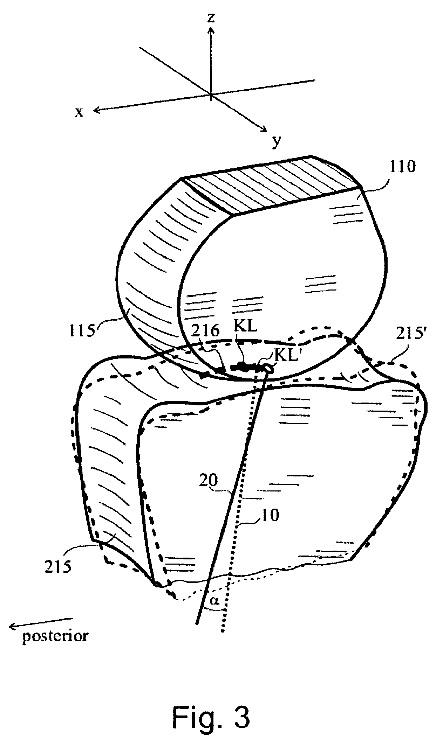

Das Roll-Gleit-Verhältnis ist als Quotient der Differentiale der Kontaktverschiebungen definiert. Dies ist in Fig. 2A anhand von aufeinander abrollenden und/oder gleitenden Kreisen illustriert. Dabei sind K1 auf dem Kreis 1 und K2 auf dem Kreis 2 jeweils diejenigen Punkte, die in der gezeigten Stellung zum momentanen Kontaktpunkt zusammenfallen. Die Differentiale der Kontaktverschiebungen sind mit $ds_{K1}$ und $ds_{K2}$ bezeichnet. Für das Roll-Gleitverhältnis gilt $\rho = ds_{K1} / ds_{K2}$ oder $\rho = ds_{K1} / ds_{K2}$ je nach Referenzsystem, wobei das Referenzsystem im Nenner steht. Dabei bedeutet $\rho = 1$ reines Rollen. Reines Gleiten des einen Teils gegenüber dem anderen wird durch $\rho = 0$, bzw. $\rho = \infty$ ausgedrückt. Roll-Gleitbewegungen haben positive Werte dazwischen. Drehen sich die Kreise um ihre Mittelpunkte gleichsinnig (d.h. die Kreisbahnen bewegen sich am Kontaktpunkt entgegengesetzt), so herrscht ebenfalls reines Gleiten, jedoch ist dann $\rho < 0$. Die Kontaktspuren müssen keine ebenen Kurven sein. Die Differentiale sind entlang der Kurven zu bilden. In Fig. 3 ist die Veränderung des lateralen Kontaktpunkts KL auf der tibial-lateralen Gelenkfläche 215 gezeigt. Die Gelenkfläche 215 ist in ihrer Lage nach Drehung durch durchgezogene Linien dargestellt. Die Gelenkfläche 215 ist gegenüber der in Fig. 1 gezeigten Streckstellung 215' der Gelenkfläche an der femoral-lateralen Gelenkfläche 115 entlang gerollt. Der ursprüngliche Kontaktpunkt der Fig. 1 ist mit KL' bezeichnet. Dabei ist KL' zum einen gestrichelt bezüglich seiner Position in der Streckstellung 215' gezeigt, und zum anderen mit durchgezogener Kontur in seiner Position nach der Drehung dargestellt. Der momentane Kontaktpunkt nach der Drehung ist mit KL bezeichnet. Auf der Oberfläche 215 ist zumindest ein Teil einer Kontaktspur 216 angedeutet, die die momentanen Kontaktpunkte beim Schwenken des Gelenks über einen Winkelbereich auf der Gelenkfläche 215 einnehmen.

Generell führt bei Ausführungsformen, die auf ein mechanisches Kniegelenk bezogen sind, dieses mechanische Kniegelenk eine Schwenkbewegung überwiegend in einer sagittalen Ebene aus. Die bei der Schwenkbewegung möglicherweise zusätzlich auftretende axiale Rotations- und/oder Ab-/Aduktions-Bewegungen sind vergleichsweise klein. Bei einem Kniegelenk ist also die Hauptfunktionsebene eine sagittale Ebene. Das ist z.B. auch bei den Fingergelenken der Fall. Bei anderen Gelenken wie z.B. Wirbeln können auch Beugungen in frontalen Ebenen erfolgen. Auch für Wirbel kann die Hauptfunktionsebene eine sagittale Ebene sein, z.B. wenn eine Vornüberbeugung betrachtet wird. Für Wirbel muss die Hauptfunktionsebene aber nicht notwendigerweise eine sagittale Ebene sein, z.B. dann nicht, wenn eine seitliche Rumpfneigung oder die axiale Rotation betrachtet wird.

Der Beugungszustand eines Kniegelenks kann von Streckung oder gar Überstreckung bis zu einer maximalen Beugung reichen. Der Streckung wird ein Schwenkwinkel von 0 Grad zugeordnet. Fig. 1 zeige den gestreckten Zustand. Im Folgenden wird zur Einfachheit der Beschreibung das Koordinatensystem der Fig. 1 mit dem Femur und dem Femurgelenkteil verbunden. Diese können dann als ortsfest angenommen werden und bleiben in ihrer Ausrichtung wie in Fig. 1 gezeigt. Dies entspricht lediglich der Wahl eines mitbewegten Koordinatensystems, nicht einem echten Festhalten des Femurs bezüglich der Umgebung. Der Schwenkwinkel oder Beugungswinkel $\alpha$ ist dann wie folgt definiert. Eine beliebige Gerade in einer sagittalen Ebene wird mit der starr Tibia verbunden. Dies kann z.B. die Gerade 10 der Fig. 1 sein, welche RL beinhaltet. Es kann aber auch jede beliebige andere Gerade genommen werden. In Fig. 3 ist die Streckstellung 215' des lateralen Tibiateils gestrichelt dargestellt. Auch die Gerade 10 der Fig. 1 ist gestreichelt gezeigt. Wird das Gelenk bewegt, so dreht sich auch die mit der Tibia starr verbundene Gerade 10 in der sagittalen Ebene. In Fig. 3 ist diese mitgedrehte Linie mit dem Zeichen 20 bezeichnet und durchgezogen dargestellt. Diese mitgedrehte Linie 20 verläuft wie zuvor durch den ursprünglichen Kontaktpunkt KL', der gegenüber seiner Lage in der Streckstellung 215' (gestrichelte Kontur) ebenfalls eine neue Lage einnimmt (durchgezogene Kontur). Der Beugungs- oder Flexionswinkel $\alpha$ ist der Winkel zwischen der Gerade 10 und ihrem mitgedrehten Äquivalent 20. Allgemein ist der Beugungs- oder Flexionswinkel $\alpha$ der Winkel zwischen einer beliebigen mit der Tibia verbundenen Gerade in Streckstellung und derselben, aber mitgedrehten Gerade. Werden als beliebige Geraden solche gewählt, die nicht in der sagittalen Ebene liegen, sondern schräg dazu, ist eine Projektion in die sagittale Ebene vorzunehmen, um den Beugungswinkel für die Beugungsbewegung bezüglich der sagittalen Ebene zu bestimmen.

In der Streckung liegen der Femur als Fortsetzung des Femurgelenkteils und die Tibia als Fortsetzung des Tibiagelenkteils im Wesentlichen auf einer Geraden. Lässt man den Femur gedanklich fest, so kann man sich den Schwenkwinkel oder Beugungswinkel α auch als Winkel zwischen der Tibia in der Streckstellung und der Tibia in der Beugungsstellung bei "festgehaltenem" Femur vorstellen.

Der Schwenkwinkel oder Beugungswinkel α kann z.B. von 0° bis 170°, typischerweise von 0° bis 160° betragen. Auch eine Überstreckung ist möglich, z.B. bis zu -1, bis zu -2, bis zu -3 Grad, bis zu -4 oder bis zu -5 Grad. Der gesamte Schwenkwinkelbereich kann z.B. von -5° bis 170°, typischerweise von -4° bis 160°, reichen.

Wird das Gelenk über seinen gesamten Schwenkwinkelbereich geführt, so beschreibt die Menge aller eingenommenen Kontaktpunkte eine Kurve auf der Gelenkfläche und eine Kurve auf der Gelenkgegenfläche. Diese Kurve wird Kontaktbereichskurve oder Kontaktspur genannt. Für das Schwenken/Beugen des mechanischen Knies ist vorwiegend der Teil der Gelenkfläche relevant, der die Kontaktspur enthält. Außerhalb kann die Gelenkfläche z.B. aufgrund anderer anatomischer Erfordernisse Formzwängen unterworfen sein, die nicht direkt mit der Gelenksfunktion zu tun haben müssen.

Die Kontaktspur kann in einer sagittalen Ebene verlaufen. Dies ist z.B. der Fall, wenn die Gelenkflächen beim Schnitt mit Frontalebenen im Bereich der Kontaktspur kreisförmig sind. In Fig. 3 verläuft beispielsweise die Kontaktspur 216 in einer sagittalen Ebene. Aber auch andere Konfigurationen sind möglich, bei der die Kontaktspur sich auch in y-Richtung (medial-lateral) verändert, z.B. zumindest abschnittsweise einen Bogen bezüglich der x-y-Ebene (Transversalebene) beschreibt.

Schnittkurven der Gelenkflächen und Gelenkgegenflächen mit sagittalen Ebenen oder mit Ebenen, die durch eine Tangente an die Kontaktspur und durch die z-Achse aufgespannt werden, sind typischerweise gekrümmt. Krümmung schließt nicht aus, dass die Schnittkurven lokal flach sein können, z.B. an einem Wendepunkt. Der Begriff "gekrümmte Fläche" und "gekrümmte Kurve" beschreibt eine globale Eigenschaft. Eine gekrümmte Fläche und eine gekrümmte Kurve können generell lokal an einem Punkt oder sogar in einem Abschnitt flach sein. Der Krümmungsradius ist in diesem Fall an besagtem Punkt oder in besagtem Abschnitt unendlich groß. Die Krümmung, als Inverses des Krümmungsradius, ist dann dort null.

Zur Bezeichnung der Krümmungsart werden die Begriffe konvex und konkav verwendet. Eine Schnittkurve ist konvex gekrümmt, wenn eine gerade Verbindung zweier Punkte der Schnittkurven innerhalb des jeweiligen Gelenkabschnitts oder -gegenabschnitts liegt, und konkav gekrümmt, wenn eine solche Verbindung außerhalb des jeweiligen Gelenkabschnitts oder -gegenabschnitts liegt. Für tibiale Gelenkkomponenten bedeutet "innerhalb" näher zur Tibia, für femurale Gelenkkomponenten bedeutet "innerhalb" näher zum Femur. Die Begriffe "konvex" und "konkav" werden auch bezüglich Abschnitten von Schnittkurven verwendet.

In Fig. 1 ist beispielsweise die Schnittkurve der Gelenkfläche 125 mit der x-z-Ebene global konvex. Die Schnittkurve der Gelenkgegenfläche 225 ist zumindest in einem mittleren Abschnitt global konkav. Gelenkfläche 115 und -gegenfläche 215 sind beide, zumindest in einem mittleren Abschnitt, konvex.

Allgemein können Schnittkurven einer Frontalebene mit einer Gelenkfläche oder Gelenkgegenfläche gekrümmt sein. Eine Frontalebene ist eine Ebene parallel zur y-z-Ebene der Fig. 1. Dabei sind die Krümmungen einer Gelenkfläche und ihrer entsprechenden Gegenfläche typischerweise gegenpässlich. Gegenpässlich bedeutet, dass die eine konvex in diesen Richtungen ist und die andere konkav. Dadurch ergibt sich eine seitliche Stabilität des mechanischen Kniegelenks, so dass der tibiale Gelenkteil 200 und der femurale Gelenkteil 100 in y-Richtung (medial-lateral) in stabiler Lage gehalten werden, weil bei einem gegeneinander "Verrutschen" wie bei einer Laufkatze ein rücktreibendes Kraftsystem entsteht. In Fig. 1 ist beispielsweise der Abschnitt 110 konvex bezüglich Schnitten mit einer Frontalebene, und der Gegenabschnitt 210 konkav. Ebenso wäre eine umgekehrte Situation denkbar.

Die Krümmungen der Gelenkflächen lassen sich durch Krümmungszentren mit zugehörigen Krümmungsradien beschreiben. Dabei wird im Folgenden die Krümmung in einer Ebene beschrieben, die von der z-Achse und der Tangente zur Kontaktspur aufgespannt wird. Ein Kreis mit dem Krümmungsradius um das Krümmungszentrum schmiegt sich in dieser Ebene an die Kontaktspur. Im Fall, dass die Kontaktspur in einer sagittalen Ebene verläuft, wird also die Krümmung in dieser sagittalen Ebene beschrieben. Die Krümmung senkrecht zur Tangente an die Kontaktspur kann positiv (konvex), negativ (konkav) oder null sein. Ist nachfolgend von "Krümmung" ohne weiteren Zusatz die Rede, so ist die Krümmung tangential zur Kontaktspur gemeint, d.h. in der Ebene aufgespannt von z-Achse und Tangente. Auch die Begriffe konvex und konkav ohne weiteren Zusatz beziehen sich auf die Krümmung in dieser Ebene. Verläuft die Kontaktspur in einer sagittalen Ebene und wird eine Gelenkfläche oder -gegenfläche als konvex oder konkav bezeichnet, so ist diese Eigenschaft bezüglich eines Schnitts mit einer sagittalen Ebene gemeint, die die Kontaktspur umfasst.

In Fig. 1 sind wie oben beschrieben die Kontaktpunkte KL und KM des Gelenks in seiner Streckstellung zu sehen. Die Kontaktspuren verlaufen bei dem mechanischen Gelenk der Fig. 1 in sagittalen Ebenen. Am Punkt KL ist die Krümmung der femoral-lateralen Gelenkfläche 110 durch das Krümmungszentrum MFL beschrieben, die Krümmung der tibial-lateralen Gelenkfläche 210 durch das Krümmungszentrum MTL. Da beide Flächen am Punkt KL konvex sind, liegen die Zentren MFL und MTL jeweils auf verschiedenen Seiten des Kontaktpunkts. Bei der femoral-medialen Gelenkfläche 120 ist die Krümmung am Kontaktpunkt KM durch das Krümmungszentrum MFM beschrieben, bei der tibial-medialen Gelenkfläche 220 durch das Krümmungszentrum MTM. Da die tibial-mediale Gelenkfläche 220 am Punkt KM konkav, die femoral-mediale Gelenkfläche 120 jedoch konvex ist, liegen die Zentren auf derselben Seite vom Kontaktpunkt KM aus.

Die Verbindung der femoralen Krümmungszentren MFM und MFL wird als Femurkoppel F bezeichnet. Die Verbindung der tibialen Krümmungszentren MTM und MTL wird als Tibiakoppel T bezeichnet. Die Verbindung der lateralen Zentren MFL und MTL wird als Lateralkoppel oder laterale Pleuel RL bezeichnet. Die Verbindung der medialen Zentren MFM und MTM wird als Medialkoppel oder mediale Pleuel RM bezeichnet.

Das mechanische Gelenk bildet in der in Fig. 1 zu sehenden Stellung ein mechanisches Viergelenk, auch viergliedriges Koppelgetriebe genannt. Charakteristisch für ein solches Viergelenk ist im Allgemeinen, dass vier bewegliche Elemente durch vier Koppeln verbunden sind, wobei jedes Element mit zwei Koppeln verbunden ist. Im Fall einer Prothese für ein Kniegelenk ist das hierin beschriebene mechanische Gelenk in einem ersten Schwenkwinkelbereich ein solches Viergelenk. Dabei bilden die "Bauteile" des Tibiagelenkteils und des Femurgelenkteils durch Kontakt der Gelenkflächen unter Kraftschluss das Viergelenk. Die kinematischen Größen werden dabei durch die Form der Gelenkflächen festgelegt. Die vier Koppeln sind durch die Femurkoppel F, Tibiakoppel T, Medialkoppel RM und Lateralkoppel RL zwischen den Krümmungszentren MFM, MFL, MTM, MTL wie oben beschrieben gegeben.

Dabei sind in räumlicher Anordnung im ersten Winkelbereich die Krümmungszentren typischerweise voneinander verschieden. Höchstens für einzelne Winkel können z.B. zwei Krümmungszentren auch identisch sein. Die Pleuel RM und RL können in parallelen sagittalen Ebenen liegen, z.B. wenn die Kontaktspuren in diesen parallelen sagittalen Ebenen liegen wie in Fig. 1 gezeigt. Die Tibiakoppel und die Femurkoppel liegen hingegen meist windschief oder schneiden sich in einem Punkt. In anderen Ausführungsformen können zwei, drei oder auch alle Koppeln windschief sein oder sich in einem Punkt schneiden. Die Pleuel RM und RL können z.B. zumindest bei einigen Flexionswinkeln oder in einigen Flexionswinkelbereichen windschief sein, wenn die Kontaktspuren nicht nur in einer sagittalen Ebene verlaufen.

Für die Diskussion der Kinematik ist oft eine Projektion der Koppeln und der Krümmungszentren in eine sagittale Ebene nützlich. Dies wird weiter unten beschrieben. Bei einer Projektion in eine sagittale Ebene wird der Begriff "Zusammenfallen" verwendet, wenn zwei Krümmungszentren in der Projektion übereinander liegen oder zu liegen kommen. Der Begriff "Auseinanderfallen" wird verwendet, wenn dies nicht der Fall ist. Beim Viergelenk fallen typischerweise die Krümmungszentren auseinander. In einigen Ausführungsformen bilden sie ein Viereck. Unter einem Viereck sei auch eine Situation verstanden, bei der drei oder gar alle vier Krümmungszentren zwar nicht zusammenfallen, jedoch in der Projektion in eine sagittale Ebene auf einer Geraden liegen (so dass die Verbindung der Punkte eher wie ein Dreieck, bzw. wie eine Gerade aussieht). In diesen Ausführungsformen soll unter einem Viereck jedoch nicht eine Entartung des Vierecks verstanden sein, die durch Zusammenfall von (mindestens zwei) Krümmungszentren entsteht.

Das mechanische Viergelenk kann auch wie in Fig. 1 zu sehen einen relativen Versatz von lateralen und medialen Gelenkabschnitten in x-Richtung (anteriorposterior) aufweisen. In Fig. 1 ist der Versatz wie auch beim menschlichen biologischen Kniegelenk dergestalt, dass der laterale Teil nach posterior versetzt ist. Ein solcher Versatz trägt dazu bei, dass - abgesehen von einer möglichen leichten Überstreckung- kein Durchschwingen der Tibia nach anterior möglich ist. Beim Kniegelenk bleibt so eine Überstreckung auf wenige Grad begrenzt.

Ein mechanisches Gelenk, das eine Viergelenksmechanik umfasst, ist aus der EP 0 617 595 bekannt. Bei diesem künstlichen Kniegelenk wird die Kinematik eines biologischen menschlichen Knies bei kleinen Flexionswinkeln gut abgebildet, d.h. bei Beugungswinkeln wie sie beim Laufen oder Gehen auftreten, z.B. Winkeln von 0° bis 30°. Bei großen Flexionswinkeln, die z.B. in der Hocke oder auch schon beim Fahrradfahren auftreten können, weicht die Kinematik jedoch erheblich vom natürlichen Verhalten ab. Insbesondere ist bei Erhalt beider Kreuzbänder das Zusammenspiel mit der Prothese bei großen Flexionswinkeln unnatürlich, wobei eine Überdehnung der Kreuzbänder zu erwarten ist und wodurch erlernte Bewegungsabläufe nicht umgesetzt werden können.

Aufgabe ist es also, ein verbessertes mechanisches Gelenk zu schaffen. Die Analyse des vorbekannten Gelenks der EP 0 617 595 zeigt, dass die Kontaktpunkte bei kleinen Flexionswinkeln nach dorsal wandern, dass es jedoch bei großen Flexionswinkeln zu einer Wanderungsumkehr kommt. Die Kontaktpunkte laufen also jenseits eines gewissen Beugungswinkels, der zu einem extremalen Kontaktpunkt führt, wieder nach anterior. Dies entspricht nicht dem natürlichen Verhalten.

Gemäß Ausführungsformen der Erfindung wird ein mechanisches Gelenk bereitgestellt, das sich in einem ersten Winkelbereich wie ein viergliedriges Koppelgetriebe und in einem zweiten Winkelbereich wie ein Scharniergelenk verhält.

Bei einem Scharniergelenk bleiben die Kontaktpunkte der Gelenkflächen auf den Gelenkgegenflächen ortsfest oder im Wesentlichen ortsfest bzw. umgekehrt die Kontaktpunkte der Gegengelenkflächen auf den Gelenkflächen ortsfest oder im Wesentlichen ortsfest. Hierbei kann "im Wesentlichen ortsfest" so verstanden werden, dass sich die Lage der Kontaktpunkte höchstens um eine Distanz verändern, die nicht mehr als 10%, typischerweise nicht mehr als 5% oder nicht mehr als 1%, der Länge der Kontaktspur beträgt. Anders kann auch Bezug zu dem Durchmesser der Gelenkabschnitte und -gegenabschnitte in anterior-posterior Richtung genommen werden. "Im Wesentlichen ortsfest" bedeutet dann, dass sich die Lage der Kontaktpunkte um nicht mehr als 10%, typischerweise nicht mehr als 5% oder nicht mehr als 1%, des Durchmessers des jeweiligen Gelenkabschnitts oder - gegenabschnitts verändert. Als Durchmesser kann die breiteste Stelle in anterior-posterior Richtung genommen werden. Bei einer Prothese für ein menschliches Kniegelenk verändert sich z.B. die Lage der Kontaktpunkte bezüglich einer sagittalen Ebene beim Verschwenken über den Scharniergelenksbereich um nicht mehr als 5 mm, typischerweise um nicht mehr als 1 mm.

Bleiben die Kontaktpunkte beim Schwenken des Kniegelenks über den zweiten Winkelbereich ortsfest oder im Wesentlichen ortsfest, so bedeutet dies ein kleines Roll-Gleit-Verhältnis. Das bedeutet, dass der Gleitanteil groß ist, oder dass sogar reines Gleiten stattfindet. Ein Scharniergelenk liegt vor, wenn das Roll-Gleit-Verhältnis kleiner als 0,1, oder sogar kleiner als 0,05 oder idealerweise sogar null ist.

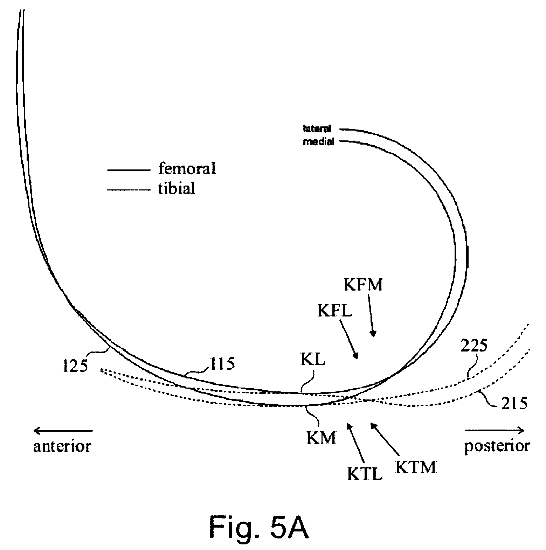

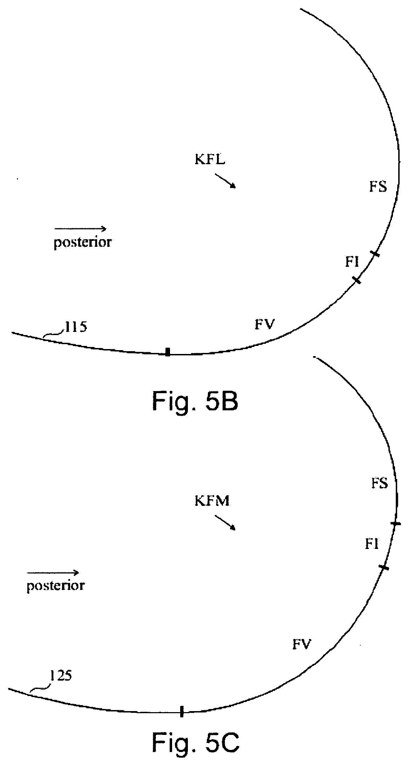

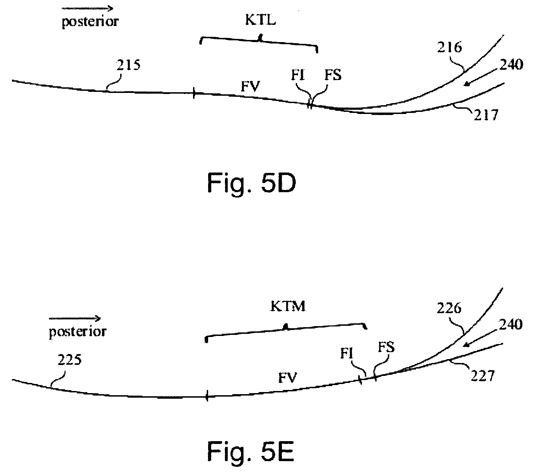

Die momentane Drehachse des mechanischen Gelenks verläuft in sagittaler Richtung, d.h. in medial-lateraler Richtung, durch den Schnittpunkt, der sich für zwei Gerade durch die Pleuel RM und RL bei Projektion in eine sagittale Ebene ergibt. Die Konstruktion wird später bezüglich der Figs. 5-8 näher beschrieben.

Im Scharniergelenksbereich des mechanischen Gelenks bleibt die momentane Drehachse des Gelenks beim Verschwenken über den Scharniergelenksbereich ortsfest oder im Wesentlichen ortsfest. Ähnlich wie oben kann "im Wesentlichen ortsfest" so verstanden werden, dass sich die Lage der momentanen Drehachse bezüglich einer sagittalen Ebene um nicht mehr als 10%, typischerweise nicht mehr als 5% oder nicht mehr als 1%, der Länge der Kontaktspur verändert. Für einen Scharniergelenksbereich z.B. von 70° bis 170° bleibt die momentane Drehachse über diesen gesamten Winkelbereich im Wesentlichen ortsfest. Anders kann auch Bezug zu dem Durchmesser der Gelenkabschnitte und -gegenabschnitte in anterior-posterior Richtung genommen werden. "Im Wesentlichen ortsfest" bedeutet dann, dass sich die Lage der Kontaktpunkte um nicht mehr als 10%, typischerweise nicht mehr als 5% oder nicht mehr als 1%, des Durchmessers des jeweiligen Gelenkabschnitts oder -gegenabschnitts verändert. Bei einer Prothese für ein menschliches Kniegelenk verändert sich z.B. die Lage der momentanen Drehachse beim Verschwenken über den Scharniergelenksbereich um nicht mehr als 5 mm, typischerweise um nicht mehr als 1 mm.

Zur Beschreibung der Veränderung/Ortsfestigkeit der Kontaktpunkte oder der momentanen Drehachse werden auch die Begriffe "Wandern" bzw. "Fixiertsein" verwendet. Einem Fixiertsein entspricht es, wenn die momentane Drehachse oder die Kontaktpunkte ortsfest oder im Wesentlichen ortsfest bleiben. Sine momentane Drehachse oder Kontaktpunkte nicht fixiert, so wird eine Veränderung der Lage auch Wandern genannt.

Bei einem Scharnier können die Krümmungszentren zweier Gelenkabschnitte oder Gelenkgegenabschnitte bezüglich einer sagittalen Ebene zusammenfallen, z.B. bezüglich der Hauptfunktionsebene. Das bedeutet, dass die Krümmungszentren übereinander liegen, wenn sie in eine sagittale Ebene projiziert werden. Die Femurkoppel F liegt dann in sagittaler Richtung senkrecht zur sagittalen Ebene, d.h. in medial-lateraler Richtung.

Der Ausdruck "bezüglich einer Ebene" oder "in Bezug auf eine Ebene" bedeutet allgemein, dass Eigenschaften der Gelenkflächen oder Gelenkgegenflächen bezüglich der Projektion in diese Ebene angegeben werden. Insbesondere können Flächenkurven wie z.B. die Kontaktspuren ggf. zusammen mit Größen wie Kontaktpunkten, Krümmungszentren, Koppeln und Pleueln in eine sagittale Ebene projiziert werden.

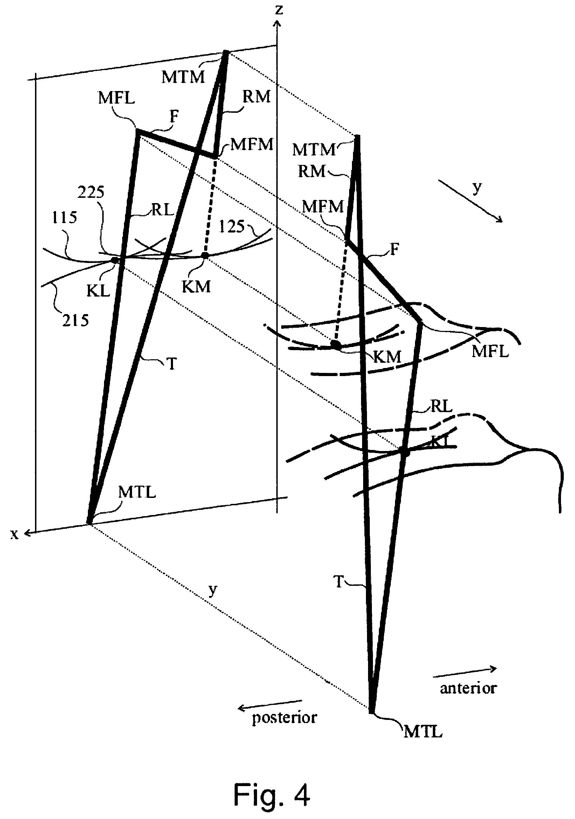

Fig. 4 illustriert eine solche Projektion. In Fig. 4 sind rechts vereinfacht die Gelenkflächen in dreidimensionaler Darstellung wie in Fig. 1 zu sehen. Die Konturen der Gelenkflächen in den sagittalen ebenen an den Kontaktpunkten sind gezeigt. Links ist eine Projektion entlang der y-Achse auf eine sagittale Ebene dargestellt. Die Figs. 5-8 zeigen solche in sagittale Ebenen projizierten Ansichten mit Kontaktspuren der einzelnen Gelenkflächen, bzw. Abschnitte davon. Die Blickrichtung in den Figs. 5-8 ist in positive y-Richtung. Eine Projektion wie in Fig. 3 links gezeigt wird also in den Figs. 5-8 "von hinten" betrachtet, so dass die posterior-Richtung nach rechts, die anterior-Richtung nach links zeigt. Die Figs. 1-4 dienen lediglich der Illustration und müssen nicht notwendigerweise Zustände eines mechanischen Gelenks gemäß Ausführungsformen der vorliegenden Erfindung zeigen.

In weiteren Ausführungsformen können auch drei Krümmungszentren im zweiten Winkelbereich, dem Scharnierbereich, zusammenfallen. Dazu müssten die Radien einer Gelenkfläche und einer Gelenkgegenfläche, z.B. der medialen, am Kontaktpunkt gleich sein und die Flächen konvex und konkav (oder konkav und konvex) gekrümmt sein. Es können weniger als vier Krümmungszentren zusammenfallen. Es können aber auch mindestens drei Krümmungszentren zusammenfallen. Z.B. könnten auch alle vier Krümmungszentren zusammenfallen. Dies wäre der Fall, wenn am Scharnierkontaktpunkt z.B. beide Gelenkflächen konvex und beide Gelenkgegenflächen konkav wären bei gleichen Krümmungsradien.

Der zweite Winkelbereich, der Scharnierbereich, kann z.B. Beugungswinkel von 35° bis 180° umfassen. Typischerweise kann der zweite Winkelbereich von 40° bis 170° Beugung betragen, noch typischer von 70° bis 170° Der erste Winkelbereich, der Viergelenksbereich, kann z.B. Beugungswinkel von 0° bis 80° betragen, typischerweise von 0° bis 70°, noch typischer von 0° bis 60°. Der erste Winkelbereich kann aber auch nur bis zu 50°, 45°, 40° oder sogar weniger betragen. Es kann auch eine Überstreckung möglich sein. In diesem Fall ist die untere Grenze des ersten Winkelbereichs statt 0° z.B. -5°, -4°, -3°, -2° oder -1°.

Zwischen dem Scharnierbereich und dem Viergelenksbereich kann ein Übergangsbereich liegen. Im Übergangsbereich kann die Kinematik von einem großen Roll-Gleit-Verhältnis, wie es sich für das Viergelenk realisieren lässt, zu einem kleinen Roll-Gleit-Verhältnis wechseln, das typisch für das Scharnier ist. Der Übergangsbereich kann z.B. Beugungswinkel von 40°, 30°, 20°, 10° oder weniger umfassen. Er kann z.B. von 40° bis 80° gehen. Gemäß einigen Ausführungsformen liegt der Übergangsbereich von 50 bis 75° oder von 60 bis 70°.

Das verbesserte mechanische Gelenk hat im ersten Winkelbereich, also für kleine Flexionswinkel wie sie z.B. im Fall eines Kniegelenks beim Gehen und Laufen auftreten, eine Viergelenksfunktion. Dabei liegt die momentane Drehachse des Gelenks wie weiter unten beschrieben nahe an den Kontaktpunkten. Das Roll-Gleit-Verhältnis kann z.B. mindestens 0,5 oder mindestens 0,6 betragen. Das Roll-Gleitverhältnis kann vom Flexionswinkel abhängen. Beispielsweise kann das Roll-Gleitverhältnis bei 5° Flexion nahezu 1 sein und bis 60° Flexion auf 60% davon absinken. Dies lässt sich durch Roll-Gleit-Kurven darstellen, d.h. durch Kurven der Roll-Gleitverhältnis-Werte als Funktion des Beugungswinkels. Das mittlere Roll-Gleitverhältnis im ersten Winkelbereich, das durch Integration ermittelt werde kann, kann z.B. mindestens 0,6, mindestens 0,7 oder mindestens 0,8 betragen. Durch den hohen Rollanteil und die Vermeidung eines hohen Gleitanteils wird im Lastfall beim Gehen oder Laufen vermieden, dass das Material der Gelenkflächen, z.B. Polyäthylen, abgerieben wird. Bei der Bewegungsumkehr, z.B. vom Vorschwenken zum Rückschwenken, treten auch Haftreibungen auf, die das Gelenkflächenmaterial bei Gleiten auf Scherung beanspruchen würden. Dies kann z.B. zum Lamellieren des Polyäthylens führen. Ein hohes Roll-Gleit-Verhältnis reduziert also auch die Beanspruchung auf Scherung für Beugungen im Viergelenksbereich. Im zweiten Winkelbereich, dem Scharnierbereich, wird die natürliche Kinematik besser nachgebildet als es eine Viergelenksmechanik gestatten würde. Erlernte Bewegungsabläufe können weiter umgesetzt werden. So sind z.B. Fahrradfahren selbst mit tiefem Sattel oder ein Hinhocken und Aufstehen möglich.

Allgemein ist jedem Beugungszustand des Gelenks ein Paar von Kontaktpunkten KM, KL jeweils auf den Gelenkflächen und -gegenflächen zugeordnet. Es bestehen mathematisch darstellbare Abbildungsvorschriften von dem gesamten Schwenkwinkelbereich A des Gelenks auf die tibialen Kontaktspuren KTM, KTL, bzw. auf die lateralen Kontaktspuren KFM, KFL, z.B.

$$f: A \rightarrow KTM \text{ x } KTL$$

$$\alpha \rightarrow (KM\_T, KL\_T)$$

und

$$g: A \rightarrow KFM \text{ x } KFL$$

$$\alpha \rightarrow (KM\_F, KL\_F)$$

wobei KM_T und KL_T die Kontaktpunkte auf den Gelenkgegenflächen der Tibia und KM_F und KL_F die Kontaktpunkte auf den Gelenkflächen des Femurs sind, die einem Beugungswinkel $\alpha$ zugeordnet sind. Bei Einnahme des Beugungswinkels $\alpha$ fallen die Punkte KM_T und KM_F zu einem medialen Kontaktpunkt KM zusammen, und die Punkte KL_T und KL_F zu einem lateralen Kontaktpunkt KL. Gemäß einigen Ausführungsformen ist die Abbildung g nicht injektiv. Insbesondere können im Scharnierbereich mehrere Winkel $\alpha$ denselben Kontaktpunkten auf den Femurgelenkflächen zugeordnet sein (z.B. reines Gleiten des Tibiagelenkteils mit unveränderten Kontaktpunkten auf dem Femurgelenkteil). In einigen Ausführungsformen ist die Abbildung g eingeschränkt auf den gesamten Schwenkwinkelbereich ohne den zweiten Winkelbereich (den Scharnierbereich) bijektiv. Mit Ausnahme der Kontaktpunkte des Scharniers ist dann eine Umkehrabbildung $g^{-1}$ definiert. Insbesondere bedeutet dies, dass es keine Bewegungsumkehr der Kontaktpunkte auf den Gelenkflächen des Femurs gibt. Die Kontaktpunkte wandern in diesen Ausführungsformen typischerweise auf den Kontaktspuren KFM und KFL kontinuierlich nach dorsal bis hin zu den Kontaktpunkten des Scharniers. Es sind auch Ausführungsformen denkbar, bei denen das für die Abbildung g Gesagte stattdessen für die Abbildung f gilt, d.h. für die Tibiagelenkflächen.

Es kann auch das axiale Drehmoment im verbesserten Viergelenk reduziert oder im Wesentlichen ausgeschaltet werden, was einen stabilen Bewegungsablauf in der Hauptfunktionsebene gestattet. Die beiden kompressiven Gelenkkräfte, deren Wirkungslinien durch die momentanen Kontaktpunkte verlaufen, können im Allgemeinen windschief zueinander sein. Das hat zur Folge, dass die beiden Kräfte in jeder Flexionsstellung ein axiales Drehmoment erzeugen, das bei kleinen Flexionswinkeln vom *M. popliteus* möglicherweise kompensiert werden kann. Das mag allerdings bei großen Flexionswinkeln nicht mehr der Fall sein, z.B. wenn der mediale Kontakt im Viergelenk schneller nach hinten wandert und den lateralen Kontakt überholt. In einem mechanischen Gelenk gemäß hierin beschriebenen Ausführungsformen können die Flächenneigungen der Gelenkflächen entlang den Kontaktkurven so gestaltet werden, dass das axiale Drehmoment Null wird bzw. nicht sein Vorzeichen wechselt.

Wird auf die Bewegung bzw. die Funktion des Gelenks abgestellt, wird daher auch der Begriff "momentaner Kontaktpunkt" verwendet. Entsprechend wird der Begriff "momentane Krümmungszentren" verwendet, wenn weniger auf die Zuordnung von Krümmungszentren zu einem Kontaktpunkt als mehr auf die Bewegung der Krümmungszentren beim Schwenken des Gelenks abgestellt wird. In einigen Ausführungsformen wandert ein Kontaktpunkt. Das bedeutet, dass sich der Kontaktpunkt einer Bewegung des Gelenks entsprechend entlang den Kontaktspuren auf beiden in Kontakt stehenden Gelenkflächen verlagert. Ein Wandern, sowohl femurseitig als auch tibiaseitig, kann z.B. dann vorliegen, wenn sich bei einer Flexion des Gelenks um 10° der Kontaktpunkt um mindestens 10% der Länge der Kontaktspur verlagert oder um mindestens 15% oder typischer noch um mindestens 20%. Dabei hängt das Ausmaß des Wanderns pro 10° Beugung vom bereits erreichten Beugungszustand ab. Wie oben beschrieben kann sich nämlich das Roll-Gleitverhältnis verändern, z.B. von nahezu 1 bei sehr kleinen Winkeln zu 0,6 bei 50-70° abnehmen. Bei einem menschlichen Kniegelenk kann ein Wandern eines Kontaktpunktes z.B. dann vorliegen, wenn sich ein Kontaktpunkt bei Flexion um 10° um 5 mm, typischer um 6 mm, noch typischer um 10 mm bewegt.

Für einen natürlichen Bewegungsablauf und zur Verminderung von Beanspruchungen der Materialien ist es vorteilhaft, den Übergang vom Viergelenk zum Scharnier kinematisch harmonisch zu gestalten. Dabei ist zu berücksichtigen, dass beim Scharniergelenk die momentane Drehachse wie oben beschrieben nahe der zwei im Wesentlichen zusammenfallenden Krümmungszentren liegt, beispielsweise nahe den femoralen Krümmungszentren MFL und MFM. Im Übergangsbereich von Viergelenk zu Scharnier wird in diesem Beispiel also die momentane Drehachse aus der Nähe der Kontaktpunkte in die Nähe der femoralen Krümmungszentren gebracht.

Hierbei bedeutet "in der Nähe", dass die momentane Drehachse von einem Krümmungszentrum, oder allgemein von einem anderen Bezugspunkt wie dem lateralen und/oder medialen Kontaktpunkt, bezüglich einer sagittalen Ebene nicht weiter als 15% der Länge der Kontaktspur, typischerweise nicht mehr als 10% der Länge der Kontaktspur entfernt ist. Bei einem menschlichen Kniegelenk kann "in der Nähe" z.B. bedeuten, dass die momentane Drehachse nicht weiter als 10 mm, typischerweise nicht weiter als 6 mm von dem Bezugspunkt entfernt liegt.

Fig. 2B illustriert an einem einfachen Beispiel einen Zusammenhang zwischen der Lage der momentanen Drehachse und dem Roll-Gleit-Verhältnis. In Fig. 2B bezeichne K den momentanen Kontaktpunkt, M1 den Mittelpunkt eines ersten Kreises mit Radius R1 und M2 den Mittelpunkt eines zweiten Kreises mit Radius R2, D die Lage der momentanen Drehachse, die im gezeigten Fall um d oberhalb des Kontaktpunktes K liege. Sei $w_1$ die Winkelgeschwindigkeit um M1 und $v_1$ die zugehörige Bahngeschwindigkeit, und sei $w_2$ die Winkelgeschwindigkeit um M2 und $v_2$ die zugehörige Bahngeschwindigkeit. Dann ist die Bahngeschwindigkeit des Punktes D null, durch den die momentane Drehachse läuft. Also gilt $(R_1-d)\cdot\omega_1+(R_2+d)\cdot\omega_2 = 0$ und, mit dem zweiten System als Referenz, für das Roll-Gleit-Verhältnis

$$\rho = \frac{v_2}{v_1} = -\frac{R_2 \cdot \omega_2}{R_1 \cdot \omega_1}.$$ Schließlich folgt daraus

$$\rho = \frac{1-d/R_1}{1+d/R_2}.$$ Man sieht also, dass eine Verkleinerung von d, wenn also d in die Nähe des Kontaktpunktes K und damit zu den Gelenkflächen hin rutscht, zu einer Erhöhung des Roll-Gleit-Verhältnisses führt. Umgekehrt, wenn sich d in Richtung von M1 bewegt, so verringert sich das Roll-Gleit-Verhältnis. Während Fig. 2B zur Anschaulichkeit die Verhältnisse an zwei kreisförmigen Gelenkflächen beschreibt, gilt Entsprechendes auch für Verallgemeinerungen auf kompliziertere Gelenkflächen, insbesondere auch auf die lateralen und medialen Gelenkflächen des mechanischen Gelenks gemäß hierin beschriebenen Ausführungsformen und für deren momentane Drehachse IRA. Es können also auch aus den angegebenen Roll-Gleit-Verhältnissen Rückschlüsse über die Bedeutung des Begriffs "in der Nähe" gewonnen werden.

Gemäß einigen Ausführungsformen ist die Krümmung mindestens einer Gelenkfläche oder mindestens einer Gelenkgegenfläche bezüglich einer sagittalen Ebene stetig. Insbesondere kann die Krümmung der Kontaktspur bezüglich einer sagittalen Ebene stetig sein. Die Krümmung wird allgemein als Inverses des Krümmungsradius zu einem gegebenen Punkt auf einer Kurve beschrieben. Wie oben beschrieben sind jedem Beugungswinkel Kontaktpunkte auf den Gelenkflächen und Gelenkgegenflächen zugeordnet, denen wiederum Krümmungsradien und damit Krümmungen bezügliche einer sagittalen Ebene zugeordnet sind. Stetigkeit der Krümmung bedeutet also, dass die Krümmungen an den Kontaktpunkten als Funktion des Beugungswinkels $\alpha$ stetig sind. Der Begriff der Stetigkeit umfasst, dass die Krümmungsfunktion nicht springt, aber Knicke aufweisen kann.

Mit Bezug zu den Figs. 5-8 werden weitere Ausführungsformen der vorliegenden Erfindung beschrieben. Dabei wird "Fig. 5" als Sammelbezeichnung für die Figs. 5A bis 5E verwendet. Die Figs. 5-8 zeigen sagittale Ansichten des mechanischen Kniegelenks, d.h. Projektionen in eine sagittale Ebene wie oben beschrieben. Speziell, jedoch ohne eine Einschränkung hierauf zu bedeuten, zeigen die Figs. 5-8 zwei überlagerte sagittale Schnitte durch die lateralen und medialen Gelenkflächen 115 und 125, bzw. Gelenkgegenflächen 215 und 225.

Dabei liegen hier die femoral-laterale Kontaktspur KFL, die femoral-mediale Kontaktspur KFM, die tibial-laterale Kontaktspur KTL und die tibial-mediale Kontaktspur KTM in den jeweiligen sagittalen Schnitten. Allgemein können Kontaktspuren auch außerhalb der sagittalen Ebenen verlaufen. Projektionen in Sagittalebenen ergeben aber dieselben Abbildungen wie die in Figs. 5-8 gezeigten.

In Fig. 5A ist die Streckstellung einer Ausführungsform des mechanischen Gelenks zu sehen. Der Flexionswinkel ist $\alpha$=0°. Die tibial-laterale Gelenkgegenfläche 215 ist hier im Kontaktbereich KTL konvex gekrümmt, die tibial-mediale Gelenkgegenfläche 225 im Kontaktbereich KTM konkav. Die femoralen Gelenkflächen 115, 125 sind in den Kontaktbereichen KFL, KFM konvex. Die Lage der Kontaktbereiche KTL, KTM, KFL und KFM werden mit Bezug zu den Figs. 5B-5E beschrieben. Die Kontaktpunkte KL und KM zwischen den lateralen und medialen Gelenkabschnitten und ihren Gegenabschnitten in Streckstellung sind in Fig. 5A gezeigt. Das Koordinatensystem ist wie in Fig. 1 orientiert. Die positive y-Richtung läuft in die Zeichenebene hinein, die positive x-Richtung nach rechts und die z-Richtung nach oben. Die lateralen und medialen Gelenkflächen dieser beispielhaften Ausführungsform sind relativ zueinander für die gezeigte Ausführungsform maßstabsgerecht, der Gesamtmaßstab kann jedoch variieren je nach Größe des mechanischen Gelenks.

Fig. 5B zeigt die femoral-laterale Gelenkfläche 115 der Fig. 5A. Eingezeichnet sind drei Bereiche FV, FI und FS, die zusammen die femoral-laterale Kontaktspur KFL ergeben. Dabei bezeichnet FV den Funktionsbereich des Viergelenks, FI einen Übergangsbereich und FS den Scharnierbereich. Die Kontaktspur KFL beginnt an der linken Markierung des Bereichs FV, die dem Kontaktpunkt für Beugungswinkel $\alpha$=0° entspricht. Das Ende im Scharnierbereich ist nicht gezeigt. Fig. 5C zeigt die femoral-mediale Gelenkfläche 125 der Fig. 5A. Eingezeichnet sind auch hier, mit gleicher Bedeutung wie in der Fig. 5B, die drei Bereiche FV, FI und FS, die zusammen die femoral-mediale Kontaktspur KFM ergeben. Die Kontaktspur KFM beginnt an der linken Markierung des Bereichs FV, die dem Kontaktpunkt für Beugungswinkel $\alpha$=0° entspricht. Das Ende im Scharnierbereich ist nicht gezeigt.

Fig. 5D zeigt die tibial-laterale Gelenkfläche 215 der Fig. 5A. Eingezeichnet sind drei Bereiche FV, FI und FS, die zusammen die tibial-laterale Kontaktspur KTL ergeben. Dabei bezeichnet FV den Funktionsbereich des Viergelenks, FI einen Übergangsbereich und FS den Scharnierbereich. Der Scharnierbereich FS besteht hier im Wesentlichen nur aus dem einen, durch die Markierung ganz rechts bezeichneten Punkt. Dieser ist bei Bewegung im Scharnierbereich der Kontaktpunkt für alle Scharnierbereichswinkel, d.h. er ist ortsfest, und gleitet über den Scharnierbereich der lateralen Kontaktspur KFL des Femurs. Die Kontaktspur KFL beginnt an der linken Markierung des Bereichs FV, die dem Kontaktpunkt für Beugungswinkel $\alpha$=0° entspricht. Das Ende der Kontaktspur KTL ist durch den Scharnierpunkt FS gegeben. Fig. 5E zeigt die tibial-mediale Gelenkfläche 225 der Fig. 5A. Eingezeichnet sind wieder die drei Bereiche FV, FI und FS, die zusammen die tibial-mediale Kontaktspur KTM ergeben. Dabei bezeichnet wieder FV den Viergelenksbereich, FI einen Übergangsbereich und FS den Scharnierbereich. Der Scharnierbereich FS besteht auch hier im Wesentlichen nur aus dem einen, durch die Markierung ganz rechts bezeichneten Punkt. Dieser ist bei Bewegung im Scharnierbereich der Kontaktpunkt für alle Scharnierbereichswinkel, d.h. er ist ortsfest, und gleitet über den Scharnierbereich der medialen Kontaktspur KFM des Femurs. Die Kontaktspur KFM beginnt an der linken Markierung des Bereichs FV, die dem Kontaktpunkt für Beugungswinkel $\alpha$=0° entspricht. Das Ende der Kontaktspur KTM ist durch den Scharnierpunkt FS gegeben.

In den Figs. 5D und 5E sind die Bereiche rechts vom jeweiligen Scharnierpunkt FS afunktionale Bereiche 240 bezüglich der Kniebeugung. Die Gelenkflächenabschnitte 217 und 227 setzen die Gelenkflächen 215 und 225 wie in der Fig. 5A gezeigt fort. Gemäß anderen Ausführungsformen setzen sich die Gelenkflächen 215, 225 außerhalb der Kontaktbereiche KTL, KTM im afunktionalen Bereich 240 mit erhöhtem Tibiaplateau fort. Dies wird durch die Gelenkflächenabschnitte 216 und 226 illustriert. Ein erhöhtes Tibiaplateau kann einer vergrößerten posterioren Stabilisierung dienen.

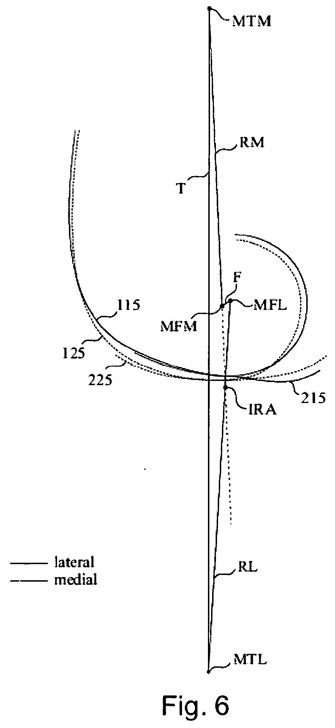

Fig. 6 zeigt dieselbe Situation des Kniegelenks in Streckstellung wie Fig. 5A. Gezeigt sind die femoralen Krümmungszentren MFM und MFL und die dazwischenliegende Femurkoppel F. Die Femurkoppel F ist in die sagittale Zeichenebene projiziert. Ihre tatsächliche Länge lässt sich daher nicht entnehmen. Zusätzlich sind die tibialen Krümmungszentren MTM und MTL gezeigt sowie die Tibiakoppel T, und die mediale Koppel RM und die laterale Koppel RT. Die die mediale Koppel RM und die laterale Koppel RL sind relativ zueinander maßstabsgerecht. Die tatsächliche Länge der Tibiakoppel T lässt sich der Figur nicht entnehmen, da die Tibiakoppel T in die Zeichenebene projiziert ist.

Die Verlängerungen der lateralen und medialen Koppeln, d.h. zwei Geraden durch die Pleuel RM und RT, schneiden sich in der Zeichenebene einem Punkt. Senkrecht zur sagittalen Zeichenebene durch diesen Schnittpunkt verläuft die momentane Drehachse IRA des Gelenks. Wie der Fig. 6 zu entnehmen liegt die momentane Drehachse IRA nahe an den momentanen Kontaktpunkten KM und KL in der Streckstellung des Gelenks. Dies bedeutet einen hohen Rollanteil beim Verschwenken des Gelenks aus der Streckstellung. Beugt man das Gelenk, so wandern die Kontaktpunkte aufgrund des Rollens nach posterior, d.h. in Fig. 6 nach rechts.

Generell kann gemäß einigen Ausführungsformen die momentane Drehachse in der Nähe mindestens eines Kontaktpunktes liegen bei einem Beugungswinkel im ersten Winkelbereich und/oder in einem Winkelbereich innerhalb des ersten Winkelbereichs und/oder im gesamten ersten Winkelbereich. Die momentane Drehachse kann auch durch mindestens einen Kontaktpunkt verlaufen. Auf der entsprechenden Gelenkfläche und Gelenkgegenfläche findet dann reines Rollen statt. Bezogen auf die Bewegung des Gelenks kann die momentane Drehachse beim Verschwenken z.B. durch einen Kontaktpunkt hindurchlaufen.

In der in Fig. 6 gezeigten Ausführungsform stellen die Koppeln F, T, RM, RL das Funktionsmodell des mechanischen Gelenks dar. In der gezeigten Konstruktion ist die Viergelenksmechanik mit hohem Rollanteil für eine Bewegung zwischen 0° und 60° Flexion maßgeblich.

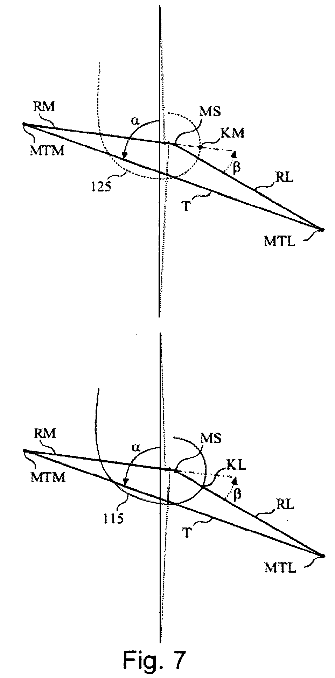

Ab einem gewissen Flexionswinkel $\alpha S$, d.h. für Flexionswinkel größer als $\alpha S$, ist eine Scharniermechanik für das mechanische Gelenk maßgeblich. Fig. 7 zeigt getrennt die mediale femorale und laterale femorale Gelenkfläche der Fig. 6, wobei zum einen die Streckstellung wie in Fig. 6 gezeigt ist und zum anderen die Situation bei einem Flexionswinkel $\alpha=70°$. In der gezeigten Ausführungsform ist auch $\alpha S=70°$. Ein Übergangsbereich von der Viergelenksmechanik zur Scharniermechanik liegt zwischen 60° und 70°.

Ein Übergangsbereich kann optional gemäß einigen Ausführungsformen der Erfindung vorliegen. Beispielsweise kann ein Übergangsbereich auf den femoralen Gelenkflächen vorgesehen sein. Bei einigen herkömmlichen Gelenken kann das Anpassen oder "Anfitten" eines Scharniergelenkbereichs an den Viergelenksbereich problematisch sein. Prinzipiell kann ab einem vorgegebenen Winkel $\alpha S$ die erreichte Position der momentanen Drehachse IRA für eine weitere Flexion, d.h. für Winkel größer als $\alpha S$, als feste Scharnierachse verwendet werden. Die Gelenkflächen können entsprechend mit zusammenfallenden Tangentenflächen gegenüber dem Viergelenksbereich fortgesetzt werden, z.B. durch kreisförmige oder nahezu kreisförmige Flächen. Jedoch können die Krümmungsradien solcher Fortsetzungen dann von den Krümmungsradien des Viergelenkbereichs stark verschieden sein. Dies kann anatomisch inakzeptabel sein. Durch einen Übergangsbereich kann durch entsprechende Gestaltung der Gelenkflächen die momentane Drehachse von einer Lage in der Nähe der Kontaktpunkte (Viergelenksbereich) in die Nähe der Krümmungszentren (Scharnier) verlegt werden. Die Krümmungsradien des sich anschließenden Scharniergelenks können dann so groß werden, dass sie mit den Krümmungsradien des Viergelenks vergleichbar sind. Dies kann anatomisch vorteilhaft sein.

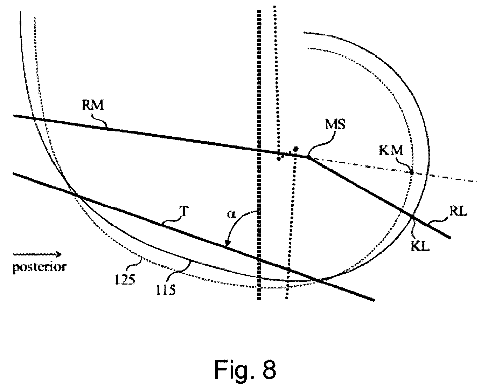

Fig. 8 zeigt einen vergrößerten Ausschnitt aus der Fig. 7, wobei laterale und mediale Gelenkfläche des Femurteils gemeinsam gezeigt sind. Die Kontaktspur lässt sich für die in Figs. 7 und 8 gezeigte Ausführungsform auf den femoralen Gelenkflächen in die drei Funktionsbereiche einteilen, die in den Figs. 5B-5E gezeigt sind. Die drei Funktionsbereiche sind der Gelenkflächenabschnitt FV mit Viergelenksfunktionalität, der Gelenkflächenabschnitt FS mit Scharnierfunktionalität, und der intermediäre Gelenkflächenabschnitt FI, der einen Übergangsbereich darstellt. Die Gelenkflächenabschnitte des Scharniers bestehen tibial im Wesentlichen aus den in Figs. 5D und 5E gezeigten Punkten FS, die sich im Scharnierbereich wie oben beschrieben nicht Wesentlich ändern. Diese tibialen Scharnierpunkte FS berühren die Femurgelenkflächen in Figs. 7 und 8 für den gezeigten Beugungswinkel $\alpha=70°$ an den gezeigten Kontaktpunkten KM und KL. Bei weiterer Drehung der Tibia und damit der Tibiakoppel T im Scharnierbereich rutschen diese ortsfesten Punkte Scharnierpunkte der Tibiaseite auf den Femurgelenkflächen entlang. Die Kontaktpunkte KM und KL verschieben sich dabei auf den Femurflächen in Figs. 7 und 8 nach oben (nach posterior). An den Kontaktpunkten sind die Krümmungen der femoralen und der lateralen Gelenkfläche so gestaltet, dass die Krümmungskreise konzentrisch sind. Das bedeutet, dass die Krümmungszentren im Punkt MS zusammenfallen und die Scharnierachse senkrecht zur Zeichenebene durch MS verläuft. Die Scharnierachse stellt die momentane Drehachse IRA für den gesamten Scharnierbereich dar.

Die Gelenkkontakte KM und KL fluchten im Scharnierbereich in der in Figs. 7 und 8 gezeigten Ausführungsform nicht. Das bedeutet, sie fallen in der Projektion nicht zusammen, sondern sind um einen Winkel $\beta$ versetzt zwischen Geraden durch die Projektionen der Pleuel RM und RL. Allgemein können gemäß Ausführungsformen der vorliegenden Erfindung im Scharnierbereich der mediale Kontaktpunkt und der laterale Kontaktpunkt verschiedene Lagen haben. Insbesondere kann die Lage auch in anterior-posterior Richtung verschieden sein, z.B. kann der laterale Kontaktpunkt dorsal, also nach posterior, gegenüber dem medialen Kontaktpunkt versetzt sein. Der Versatz kann gemäß einigen Ausführungsformen beispielsweise mindestens 1 %, mindestens 5% oder mindestens 10% der Länge der längsten Kontaktspur oder der des größten Durchmessers eines der Gelenkteile betragen. Der Versatz kann beispielsweise höchstens 20 %, höchstens 10% oder höchstens 5% der Länge der längsten Kontaktspur oder der des größten Durchmessers eines der Gelenkteile betragen. Bei einem menschlichen Knie kann der Versatz beispielsweise von 0,3 mm bis 10 mm, von 1 mm bis 8 mm oder von 2 mm bis 6 mm betragen. Der Versatz kann von Länge und Position der Funktionsbereiche (Viergelenksbereich, Übergangsbereich, Scharnierbereich) abhängen. Es können sich auch andere Werte für den Versatz ergeben. Ein Versatz der Kontaktpunkte im Scharnierbereich kann es z.B. erleichtern, eine stabile Hocke einzunehmen und aus einer solchen Hocke wieder aufzustehen. Der muskuläre Apparat kann in diesem Fall einen Wechsel des Gleichgewichts zwischen mechanisch stabilem Zustand (Hockstellung) und instabilem Zustand (Aufstehen) einstellen.

In der in Figs. 7 und 8 dargestellten Ausführungsform sind die tibialen Gelenkflächen, die nicht gezeigt sind, kreisförmig oder nahezu kreisförmig zumindest im Kontaktbereich, d.h. entlang der Länge der Kontaktspur (siehe z.B. Figs 5 und 6). Eine solche Gestaltung ist einfach in der Herstellung. Jedoch können gemäß anderen Ausführungsformen auch allgemeinere tibiale Gelenkflächen verwendet werden. Als Konsequenz der Kreisförmigkeit ergibt sich, dass die Tibiakoppel T von gleicher Länge bleibt und nur gedreht wird, wenn das Tibiagelenkteil relativ zum ortsfest gedachten Femurgelenkteil schwenkt. In der speziellen Ausführungsform der Figs. 7 und 8 kann daher der Flexionswinkel α zwischen der Tibiakoppel T' der Streckstellung und der Tibiakoppel T der gebeugten Stellung des Gelenks abgelesen werden.

Die Ausführungsformen der vorliegenden Erfindung, die mit Bezug zu den Figs. 5-8 beschrieben worden sind, können z.B. Weiterentwicklungen der AEQUOS-Kniegelenksprothese umfassen. Solche AEQUOS-Prothesen der zweiten Generation können z.B. gegenüber der ersten Generation ein unverändertes Tibiagelenkteil umfassen. Die erprobt guten tribologischen Werte der Tibiagelenkflächen blieben erhalten. Das Scharniergelenk im zweiten Winkelbereich würde durch Veränderung der femoralen Gelenkflächen realisiert. Beispielsweise umfassen die Ausführungsformen eine Weiterentwicklung, bei der die Krümmungen der femoralen Gelenkflächen vom Viergelenksbereich über einen Übergangsbereich zum Scharnierbereich stetig verändert werden.

Gemäß weiteren Ausführungsformen, kann das mechanische Viergelenk mindestens ein Merkmal aus der nachfolgenden Gruppe von Merkmalen umfassen: die femoralen Krümmungsradien betragen von 10 mm bis 40 mm, z.B. von 15 mm bis 30 mm oder von 20 mm bis 25 mm; die tibialen Krümmungsradien betragen von 20 mm bis 90 mm, z.B. von 30 mm bis 80 mm oder von 40 mm bis 70 mm; die tibial-medialen Krümmungsradien der Gelenkgegenflächen sind größer als die entsprechenden femoral-medialen Krümmungsradien der Gelenkflächen (dies kann auch für die entsprechenden femoralen Krümmungsradien gelten); die tibial-medialen Krümmungen der Gelenkflächen sind kleiner als die femoral-medialen Krümmungen der Gelenkgegenflächen (dies kann auch für die entsprechenden femoralen Krümmungsradien gelten); die Gelenkflächen, bzw. Gelenkgegenflächen umfassen oder bestehen aus mindestens einem der Materialien Polyäthylen (insbesondere Tibigelenkgegenflächen), Stahl, z.B. Chrom-Mangan-Stahl (insbesondere Femurgelenkflächen), Titan, Keramik, PEEK und Kombinationen davon.

**Patentansprüche**

1. Mechanisches Gelenk, insbesondere Endoprothese für ein menschliches Gelenk, umfassend einen ersten Gelenkabschnitt (110) mit einer ersten Gelenkfläche (115), einen ersten Gelenkgegenabschnitt (210) mit einer ersten Gelenkgegenfläche (215), einen zweiten Gelenkabschnitt (120) mit einer zweiten Gelenkflächen (125) und einen zweiten Gelenkgegenabschnitt (220) mit einer zweiten Gelenkgegenfläche (225), wobei die beiden Gelenkabschnitte (110, 120) zu einem ersten Gelenkteil (100) und die beiden Gelenkgegenabschnitte (210, 220) zu einem zweiten Gelenkteil (200) starr verbunden sind,
und wobei das Gelenk für ein menschliches Kniegelenk ist und die erste Gelenkfläche (115) femoral-lateral, die erste Gelenkgegenfläche (215) tibial-lateral, die zweite Gelenkfläche (125) femoral-medial und die zweite Gelenkgegenfläche (225) tibial-medial liegt,
wobei die erste Gelenkfläche (115) und die erste Gelenkgegenfläche (215) durch Berührung an einem ersten Kontaktpunkt eine gelenkige erste Verbindung bilden und die zweite Gelenkfläche (125) und die zweite Gelenkgegenfläche (225) durch Berührung an einem zweiten Kontaktpunkt eine gelenkige zweite Verbindung bilden, so dass der erste Gelenkteil (100) relativ zum zweiten Gelenkteil (200) eine Schwenkbewegung in einer Hauptfunktionsebene durchführen kann,
wobei in Bezug auf die Hauptfunktionsebene die Stärken der Krümmungen der ersten Gelenkfläche (115) und der ersten Gelenkgegenfläche (215) am ersten Kontaktpunkt durch erste Krümmungszentren und die Stärken der Krümmungen der zweiten Gelenkfläche (125) und der zweiten Gelenkgegenfläche (225) am zweiten Kontaktpunkt durch zweite Krümmungszentren beschrieben werden,
**dadurch gekennzeichnet, dass**
innerhalb der Hauptfunktionsebene für Schwenkwinkel in einem ersten Winkelbereich die Krümmungszentren auseinanderfallen, wobei sie typischerweise ein Viereck bilden, und für Schwenkwinkel in einem zweiten Winkelbereich mindestens zwei der Krümmungszentren zusammenfallen, und
wobei die beiden Gelenkteile (100, 200) für Schenkwinkel in dem ersten Winkelbereich ein viergliedriges Koppelgetriebe und für Schwenkwinkel in dem zweiten Winkelbereich ein Scharniergelenk bilden.

2. Mechanisches Gelenk nach Anspruch I, wobei im zweiten Winkelbereich weniger als vier der Krümmungszentren bezüglich der Hauptfunktionsebene zusammenfallen.

3. Mechanisches Gelenk nach einem der Ansprüche 1-2, wobei im zweiten Winkelbereich bezüglich der Hauptfunktionsebene die Krammungszentren der beiden Gelenkflächen, (115, 125) zusammenfallen und/oder die Krümmungszentren der beiden Gelenkgegenflächen (215, 225) zusammenfallen.

4. Mechanisches Gelenk nach einem der Ansprüche 1-3, wobei für den ersten Winkelbereich sich bezüglich der Hauptfunktionsebene eine Verbindungsgerade zwischen den momentanen Krümmungszentren der Gelenkflächen (115, 125) und eine Verbindungsgerade zwischen den momentanen Krümmungszentren der Gelenkgegenflächen (215, 225) schneiden.

5. Mechanisches Gelenk nach einem der Ansprüche 1-4, wobei für den zweiten Schwenkwinkelbereich die beiden Kontaktpunkte in der Hauptfunktionsebene versetzt liegen.

6. Mechanisches Gelenk nach einem der vorherigen Ansprüche, wobei das Roll-Gleit-Verhältnis der Schwenkbewegung im ersten Winkelbereich größer als 0,5 und im zweiten Winkelbereich kleiner als 0,1 ist.

7. Mechanisches Gelenk nach einem der vorherigen Ansprüche, wobei die momentane Drehachse der Schwenkbewegung für Schwenkwinkel im zweiten Winkelbereich im Wesentlichen fixiert ist und für Schwenkwinkel im ersten Winkelbereich in der Hauptfunktionsebene wandert.

8. Mechanisches Gelenk nach Anspruch 7, wobei die momentane Drehachse bei einer kontinuierlichen Schwenkbewegung vom ersten zum zweiten Winkelbereich kontinuierlich wandert.

9. Mechanisches Gelenk nach einem der Ansprüche 1-8, wobei die beiden Kontaktpunkte für Schwenkwinkel im zweiten Winkelbereich im Wesentlichen fixiert sind und für Schwenkwinkel im ersten Winkelbereich in der Hauptfunktionsebene wandern.

10. Mechanisches Gelenk nach einem der Ansprüche 1-9, wobei bezüglich der Hauptfunktionsebene die erste Gelenkfläche (115) einen konvex geformten ersten Flächenabschnitt, die zweite Gelenkfläche (125) einen konvex geformten zweiten Flächenabschnitt, die erste Gelenkgegenfläche (215) einen konvex geformten ersten Gegenflächenabschnitt und die zweite Gelenkgegenfläche (225) einen konkav geformten zweiten Gegenflächenabschnitt aufweist.

11. Mechanisches Gelenk nach Anspruch 10, wobei die erste Gelenkgegenfläche (215) einen konkav geformten weiteren Gelenkflächenabschnitt aufweist.

12. Mechanisches Gelenk nach einem der vorherigen Ansprüche, wobei der erste Winkelbereich mindestens 20° umfasst, beispielsweise mindestens 40° oder bis zu 60° oder bis zu 70°.

13. Mechanisches Gelenk nach einem der Ansprüche 1-12, wobei die Krümmung mindestens einer der Gelenkflächen (115, 125) und Gegengelenkflachen (215, 225) in der Hauptfunktionsebene stetig ist.

14. Mechanisches Gelenk nach einem der Ansprüche 1-13, wobei es einen dritten Winkelbereich gibt, bei dem die erste Gelenkfläche (115) und erste Gelenkgegenfläche (215) und/oder die zweite Gelenkfläche (125) und zweite Gelenkgegenfläche (225) bezüglich der Hauptfunktionsebene inkongruent sind.

15. Mechanisches Gelenk nach einem der Ansprüche 1-14, wobei die momentane Drehachse in der Hauptfunktionsebene beim Verschwenken im ersten Winkelbereich durch die Nähe mindestens eines Kontaktpunkts läuft.

**Claims**

1. Mechanical joint, in particular an endoprosthesis for a human joint, comprising a first joint section (110) having a first joint surface (115), a first joint counter-section (210) having a first joint counter-surface (215), a second joint section (120) having a second joint surface (125), and a second joint counter-section (220) having a second joint counter-surface (225),
   wherein the two joint sections (110, 120) are rigidly connected to form a first joint part (100), and the two joint counter-sections (210, 220) are rigidly connected to form a second joint part (200),
   and wherein the joint is intended for a human knee joint, and
   the first joint surface (115) is located femoral-laterally, the first joint counter-surface (215) tibial-laterally, the second joint surface (125) femoral-medially, and the second joint counter-surface (225) tibial-medially,
   wherein the first joint surface (115) and the first joint counter-surface (215) form an articulated first connection by a contact at a first contact point, and the second joint surface (125) and the second joint counter-surface (225) form an articulated second connection by a contact at a second contact point, so that the first joint part (100) may perform a pivoting movement relative to the second joint part (200) in a main functional plane,
   wherein with respect to the main functional plane, the extents of the curvatures of the first joint surface (115) and the first joint counter-surface (215) at the first contact point are described by first centres of curvature, and the extents of the curvatures of the second joint surface (125) and the second joint counter-surface (225) at the second contact point are described by second centres of curvature, **characterized in that**
   the centres of curvature fall apart within the main functional plane for pivoting angles in a first angular region, while they typically form a quadrangle, and at least two of the centres of curvature coincide for pivoting angles in a second angular region, and

wherein the two joint parts (100, 200) form a four-termed coupler mechanism for pivoting angles in the first angular region, and form a hinge joint for pivoting angles in the second angular region.

2. Mechanical joint according to claim 1, wherein in the second angular region, less than four of the centres of curvature coincide with respect to the main functional plane.

3. Mechanical joint according to any of claims 1 to 2, wherein in the second angular region, the centres of curvature of the two joint surfaces (115, 125) coincide, and/or the centres of curvature of the two joint counter-surfaces (215, 225) coincide with respect to the main functional plane.

4. Mechanical joint according to any of claims 1 to 3, wherein for the first angular region, a connecting straight line between the current centres of curvature of the joint surfaces (115, 125) and a connecting straight line between the current centres of curvature of the joint counter-surfaces (215, 225) intersect with respect to the main functional plane.

5. Mechanical joint according to any of claims 1 to 4, wherein for the second pivoting angular region, the two contact points are lying offset with respect to each other in the main functional plane.

6. Mechanical joint according to any of the preceding claims, wherein the rolling/sliding ratio of the pivoting movement is higher than 0.5 in the first angular region, and lower than 0.1 in the second angular region.

7. Mechanical joint according to any of the preceding claims, wherein the current axis of rotation of the pivoting movement is essentially fixed for pivoting angles in the second angular region, and is travelling for pivoting angles in the first angular region in the main functional plane.

8. Mechanical joint according to claim 7, wherein the current axis of rotation is continuously travelling from the first to the second angular region at a continuous pivoting movement.

9. Mechanical joint according to any of claims 1 to 8, wherein the two contact points for pivoting angles in the second angular region are essentially fixed, and travel in the main functional plane for pivoting angles in the first angular region.

10. Mechanical joint according to any of claims 1 to 9, wherein with respect to the main functional plane, the first joint surface (115) comprises a convexly shaped first surface section, the second joint surface (125) comprises a convexly shaped second surface section, the first joint counter-surface (215) comprises a convexly shaped first counter-surface section, and the second joint counter-surface (225) comprises a concavely shaped second counter-surface section.

11. Mechanical joint according to claim 10, wherein the first joint counter-surface (215) comprises a concavely shaped further joint surface section.

12. Mechanical joint according to any of the preceding claims, wherein the first angular region includes at least 20°, for example at least 40° or up to 60° or up to 70°.

13. Mechanical joint according to any of claims 1 to 12, wherein the curvature of at least one of the joint surfaces (115, 125) and joint counter-surfaces (215, 225) is continual in the main functional plane.

14. Mechanical joint according to any of claims 1 to 13, wherein there is a third angular region in which the first joint surface (115) and the first joint counter-surface (215), and/or the second joint surface (125) and the second joint counter-surface (225) are incongruent with respect to the main functional plane.

15. Mechanical joint according to any of claims 1 to 14, wherein the current axis of rotation in the main functional plane passes through the vicinity of at least one contact point while it pivots in the first angular region.

**Revendications**

1. Articulation mécanique, en particulier endoprothèse pour une articulation humaine, comprenant une première section d'articulation (110) dotée d'une première surface d'articulation (115), une première section complémentaire d'articulation (210) dotée d'une première surface complémentaire d'articulation (215), une seconde section d'articulation (120) dotée d'une seconde surface d'articulation (125) et une seconde section complémentaire d'articulation (220) dotée d'une seconde surface complémentaire d'articulation (225), les deux sections d'articulation (110, 120) étant reliées rigidement en une première partie d'articulation (100) et les deux sections complémentaires d'articulation (210, 220) en une seconde partie d'articulation (200),
et l'articulation étant destinée à une articulation du genou humaine et la première surface d'articulation (115) étant fémorale latérale, la première surface complémentaire d'articulation (215) tibiale latérale, la seconde surface d'articulation (125) fémorale médiale et la seconde surface complémentaire d'articulation (225) tibiale médiale,
la première surface d'articulation (115) et la première surface complémentaire d'articulation (215) formant une première liaison articulée par le contact sur un premier point de contact et la seconde surface d'articulation (125) et la seconde surface complémentaire d'articulation (225) formant une seconde liaison articulée par le contact sur un second point de contact, de telle sorte que la première partie d'articulation (100) peut effectuer un mouvement de pivotement par rapport à la seconde partie d'articulation (200) dans un plan de fonction principal,
les intensités des courbures de la première surface d'articulation (115) et de la première surface complémentaire d'articulation (215) étant décrites sur le premier point de contact par des premiers centres de courbure et les intensités des courbures de la seconde surface d'articulation (125) et de la seconde surface complémentaire d'articulation (225) sur le second point de contact par des seconds centres de courbure, par rapport au plan de fonction principal, **caractérisée en ce que**
dans le plan de fonction principal, les centres de courbure divergent pour des angles de pivotement dans une première plage angulaire, formant typiquement un quadrilatère, et au moins deux des centres de courbure coïncident pour des angles de pivotement dans une seconde plage angulaire, et
les deux parties d'articulation (100, 200) formant un mécanisme articulé à quatre membres pour des angles de pivotement dans la première plage angulaire et une articulation en charnière pour des angles de pivotement dans la seconde plage angulaire.

2. Articulation mécanique selon la revendication 1, dans laquelle dans la seconde plage angulaire moins de quatre des centres de courbure coïncident par rapport au plan de fonction principal.

3. Articulation mécanique selon l'une des revendications 1 - 2, dans laquelle dans la seconde plage angulaire, les centres de courbure des deux surfaces d'articulation (115, 125) coïncident et/ou les centres de courbure des deux surfaces complémentaires d'articulation (215, 225) coïncident, par rapport au plan de fonction principal.

4. Articulation mécanique selon l'une des revendications 1 - 3, dans laquelle pour la première plage angulaire, une droite de liaison entre les centres de courbure momentanés des surfaces d'articulation (115, 125) et une droite de liaison entre les centres de courbure momentanés des surfaces complémentaires d'articulation (215, 225) se croisent par rapport au plan de fonction principal.

5. Articulation mécanique selon l'une des revendications 1 - 4, dans laquelle pour la seconde plage angulaire de pivotement, les deux points de contact sont décalés dans le plan de fonction principal.

6. Articulation mécanique selon l'une des revendications précédentes, dans laquelle le rapport roulement-glissement du mouvement de pivotement est supérieur à 0,5 dans la première plage angulaire et inférieur à 0,1 dans la seconde plage angulaire.

7. Articulation mécanique selon l'une des revendications précédentes, dans laquelle l'axe de rotation momentané du mouvement de pivotement pour des angles de pivotement dans la seconde plage angulaire est essentiellement fixe et se déplace dans le plan de fonction principal pour des angles de pivotement dans la première plage angulaire.

8. Articulation mécanique selon la revendication 7, dans laquelle l'axe de rotation momentané se déplace en continu lors d'un mouvement de pivotement continu de la première à la seconde plage angulaire.

9. Articulation mécanique selon l'une des revendications 1 - 8, dans laquelle les deux points de contact pour des angles de pivotement dans la seconde plage angulaire sont essentiellement fixes et se déplacent dans le plan de fonction principal pour des angles de pivotement dans la première plage angulaire.

10. Articulation mécanique selon l'une des revendications 1 - 9, dans laquelle, par rapport au plan de fonction principal, la première surface d'articulation (115) comporte une première section de surface de forme convexe, la seconde surface d'articulation (125) une seconde section de surface de forme convexe, la première surface complémentaire d'articulation (215) une première section de surface complémentaire de forme convexe et la seconde surface complémentaire d'articulation (225) une seconde section de surface complémentaire de forme concave.

11. Articulation mécanique selon la revendication 10, dans laquelle la première surface complémentaire d'articulation (215) comporte une autre section de surface d'articulation de forme concave.

12. Articulation mécanique selon l'une des revendications précédentes, dans laquelle la première plage angulaire comprend au moins 20°, par exemple au moins 40° ou jusqu'à 60° ou jusqu'à 70°.

13. Articulation mécanique selon l'une des revendications 1 à 12, dans laquelle la courbure d'au moins une des surfaces d'articulation (115, 125) et des surfaces complémentaires d'articulation (215, 225) est régulière dans le plan de fonction principal.

**14.** Articulation mécanique selon l'une des revendications 1 - 13, dans laquelle il y a une troisième plage angulaire, pour laquelle la première surface d'articulation (115) et la première surface complémentaire d'articulation (215) et/ou la seconde surface d'articulation (125) et la seconde surface complémentaire d'articulation (225) sont non congruentes par rapport au plan de fonction principal.

**15.** Articulation mécanique selon l'une des revendications 1 - 14, dans laquelle l'axe de rotation momentané dans le plan de fonction principal passe à proximité d'au moins un point de contact lors du pivotement dans la première plage angulaire.

Fig. 1

Fig. 2A

Fig. 2B

Fig. 3

Fig. 4

Fig. 5A

KFL

posterior

FS

FI

FV

115

## Fig. 5B

KFM

posterior

FS

FI

FV

125

## Fig. 5C

posterior

KTL

215

216

240

FI    FS

FV

217

## Fig. 5D

posterior

KTM

225

226

240

FI    FS

FV

227

## Fig. 5E

MTM

RM

T

F

MFM    MFL

115

125

225

IRA    215

———— lateral
– – – – medial

RL

MTL

Fig. 6

24

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0617595 A